# EUROPEAN PATENT SPECIFICATION

(11) **EP 2 192 874 B1**
(45) Date of publication and mention of the grant of the patent: **02.01.2013**
(21) Application number: 08828552.3
(22) Date of filing: 21.08.2008
(51) Int. Cl.: A61F 2/16

(54) **INTRAOCULAR LENS PACKAGING**
VERPACKUNG FÜR INTRAOKULARLINSEN
CONDITIONNEMENT DE LENTILLE INTRAOCULAIRE

(30) Priority: 30.08.2007 US 847398
(43) Date of publication of application: 09.06.2010
(73) Proprietor: Bausch & Lomb Incorporated, Rochester, NY 14604-2701 (US)
(72) Inventor: RATHERT, Brian, D., Largo, FL 33778 (US); BESSIERE, Benoit, 31280 Dremil Lafage (FR); WAGNER, Christopher, E., Webster, NY 14580 (US); AYYAGARI, Madhu, Fairport, NY 14450 (US)
(74) Representative: Vossius & Partner
(86) International application number: PCT/US2008/073822
(87) International publication number: WO 2009/029472

(56) References cited:
- EP-A- 1 042 999
- US-A- 4 257 521
- US-A1- 2002 029 981
- US-B1- 6 183 513

## Description

### Field of Invention

The present invention relates to containers for holding intraocular lenses (IOLs), and more particularly to containers for maintaining an IOL in a liquid.

### Background of the Invention

IOLs are artificial lenses used to replace natural crystalline lenses of patients' when their natural lenses are diseased or otherwise impaired. Under some circumstances a natural lens may remain in a patient's eye together with an implanted IOL. IOLs may be placed in either the posterior chamber or the anterior chamber of an eye. IOLs come in a variety of configurations and materials (e.g., hydrophilic materials or hydrophobic materials). Various inserter instruments and methods for implanting such IOLs in an eye are known. Typically, an incision is made in a patient's cornea and an IOL is inserted into the eye through the incision. In one technique, a surgeon uses surgical forceps to grasp the IOL and insert it through the incision into the eye. While this technique is still practiced today, more and more surgeons are using IOL injectors, which offer advantages such as affording a surgeon more control when inserting an IOL into an eye and permitting insertion of IOLs through smaller incisions.

IOLs are packaged and presented to an operating room facility in a primary package (i.e. in a package capable of maintaining an IOL in a sterile state). Configuration of a package in a manner that facilitates removal and readying for use with an inserter is important to efficient, clean, and accurate IOL insertion.

IOLs made of hydrophylic materials (e.g., hydrophilic acrylics) present a particular issue during loading the IOL. The liquid (e.g., saline) in which the IOL is packaged tends to be difficult to control. Spillage or leakage or, general, a lack of control of liquid matter during loading and insertion process may cause a surgeon reduced tactile control or other consternation. An IOL package according to the preamble of claim 1 is known from the document US - B- 6183513.

### Summary

Aspects of the present invention are directed to an IOL package that provides increased liquid control. In particular, the package is constructed in a manner such that a gripping surface on an IOL holder is disposed so as to be maintained in a sterile state, yet protected from the liquid.

Aspects of the invention are directed to an IOL package, comprising a container comprising a first surface containing a liquid inside, a holder comprising a second surface and a handle with a gripping surface, an IOL held inside said container by said holder, and a lid stock attached to said first surface and said second surface, so as to form a first liquid barrier with said container and a second liquid barrier with said holder, whereby the gripping surface is protected from the liquid.

The holder comprises a recess and said handle is formed in said recess. The first liquid barrier may be a sterile barrier. The handle is disposed under the lid stock.

The IOL may be directly connected to the holder or the IOL may be coupled to the holder by one or more intermediary components. In some embodiments, the one or more intermediary components comprise a shuttle having a lumen extending therethrough. The IOL may be disposed in the shuttle in an unstressed state.

### Brief Description of the Drawings

Illustrative, non-limiting embodiments of the present invention will be described by way of example with reference to the accompanying drawings, in which the same reference number is used to designate the same or similar components in different figures, and in which:
FIG. 1 is a schematic illustration of an example of an embodiment of an IOL package according to aspects of the present invention;
FIG. 2 is a schematic illustration of the IOL package of FIG. 1 in which a foil cover has been removed;
FIG. 3 is a schematic illustration of the IOL package of FIG. 1 in which the holder with the IOL coupled thereto is removed from the container;
FIG. 4 is a schematic illustration of the IOL package of FIG. 1 showing further details of the lid stock connections to the container and holder;
FIG. 5 is a cutaway cross section view of the IOL package of FIG. 1 with a holder holding a shuttle with an IOL disposed in the shuttle;
FIG. 6 is a schematic illustration showing the shuttle of FIG. 1 being loaded into an IOL injector;
FIG. 7 is a schematic illustration showing the shuttle of FIG. 1 in greater detail; and
FIG. 8 is a schematic view of another example of an embodiment of an IOL holder for use with IOL packages according to aspects of the invention.

### Detailed Description

Aspects of the present invention are directed to an IOL package comprising elements as follows: a container including a first surface and containing a liquid inside, a holder including a second surface and a handle with a gripping surface, an IOL held inside said container by said holder, and a lid stock. The lid stock is attached to said first surface and said second surface so as to form a liquid barrier with both said container and said holder. The gripping surface is disposed so as to be protected from the liquid.

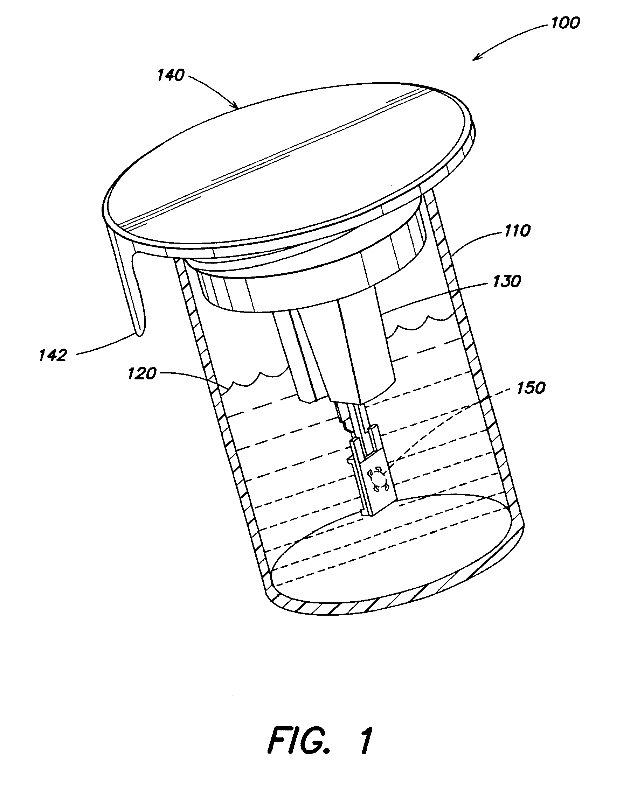

FIG. 1 is a schematic illustration of an example of an embodiment of an IOL package 100 according to aspects of the present invention. The IOL package comprises a container 110 with liquid 120 inside, a holder 130, and a lid stock 140 (with a pull tab 142). As is discussed in greater detail below, the holder holds an IOL 150 inside the container.

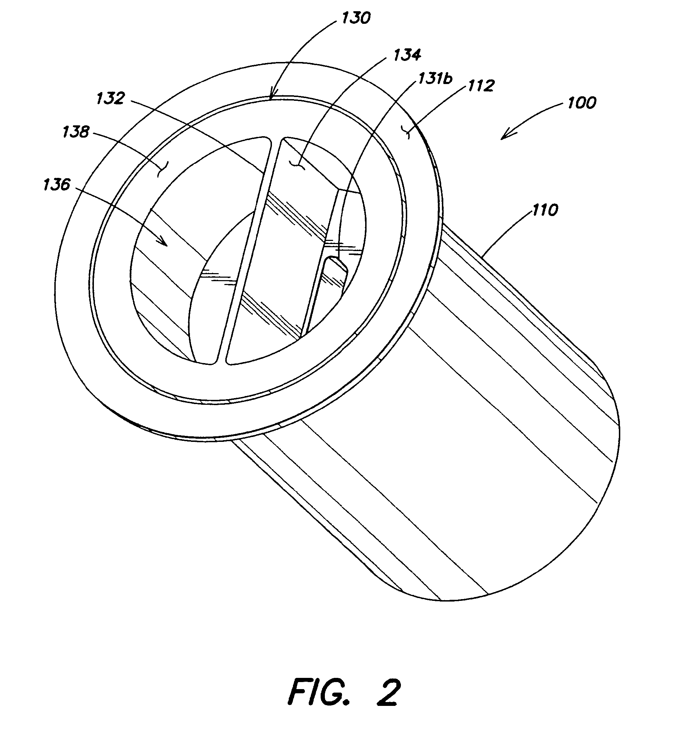

FIG. 2 is a schematic illustration of IOL package 100 of FIG. 1 in which the lid stock has been removed to facilitate viewing of holder 130. As shown in FIG. 2, holder 130 has a handle 132 (with a gripping surface 134) for lifting holder 130 and IOL 150 from container 110. The holder includes a recess 136; and handle 132 is formed in said recess. The recess and handle are configured to facilitate human fingers (not shown) extending into portions of the recess to grab the handle and remove the holder and IOL from the container.

As is described in greater detail below, according to aspects of the present invention, the container comprises a first surface 112 and the holder comprises a second surface 138. The lid stock (shown in FIG. 1) is connected to each of the first surface and the second surface, so as to form a liquid barrier with each surface. The first surface and the second surface and the corresponding barriers (i.e., seals) formed with the lid stock may be round (as shown), oval, polygonal or any other suitable shape.

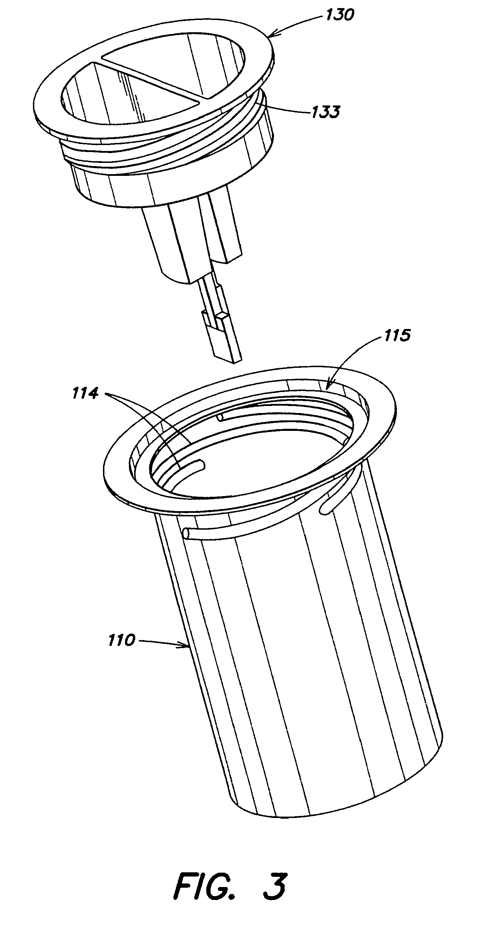

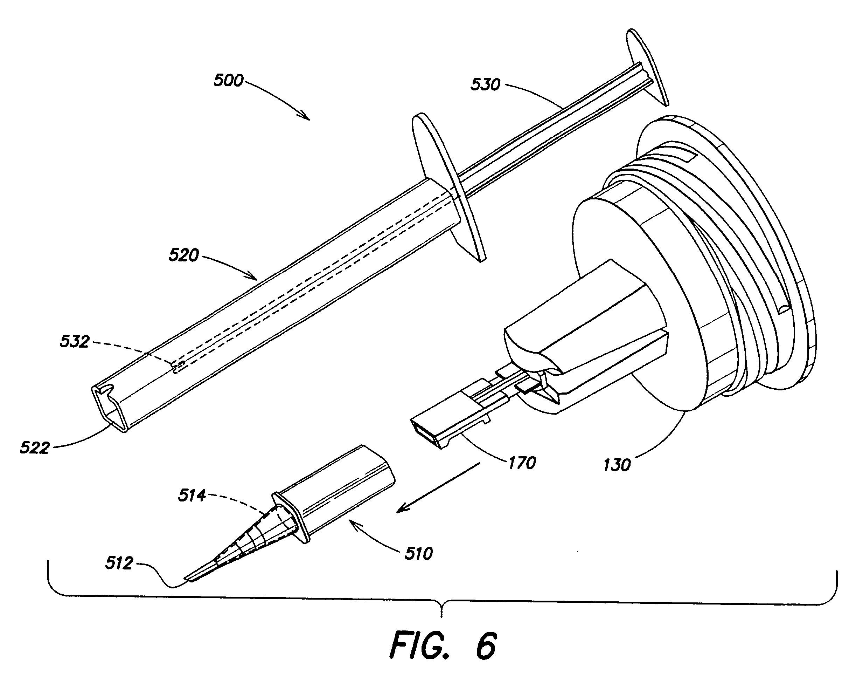

FIG. 3 is a schematic illustration of the IOL package of FIG. 1 in which holder 130, with the IOL coupled thereto, is removed from container 110. It will be appreciated that a member of a surgical staff can remove the lens (using handle 132) and load the lens into an inserter (shown in FIG. 6) while reducing or eliminating the likelihood of getting liquid 120 on his or her fingers. Referring again to FIG. 3, the container has an opening 115 through which the container may be filled with the liquid and through which the IOL is placed into and removed from the container.

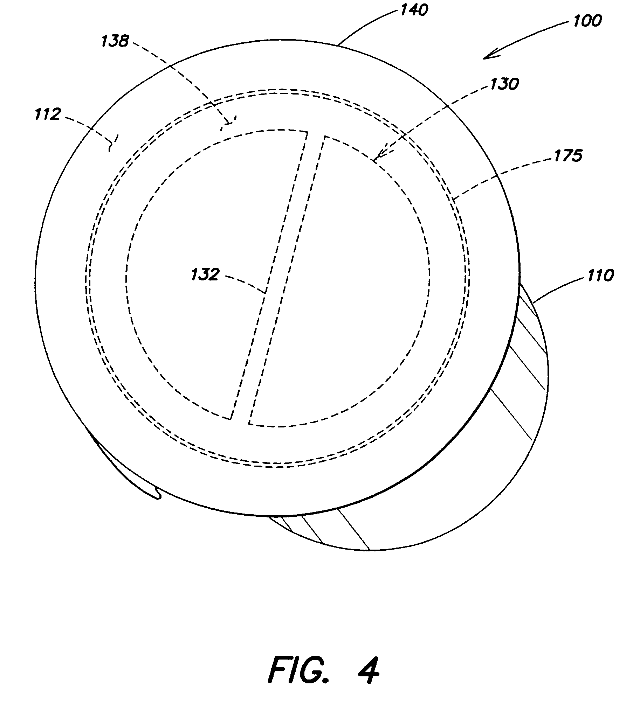

FIG. 4 is a top view of IOL package 100 of FIG. 1 showing further details of lid stock 140 connections to the container 110 and holder 130. FIG. 4 shows the first surface 112 and the second surface 138 through the lid stock. The lid stock may be connected to the container across all or a portion of surface 112 and may connected to the holder across all or a portion of surface 138.

In the fully packaged state, the lid stock is attached to the first surface and the second surface, so as to form a first liquid barrier with said container and a second liquid barrier with said holder. It will be appreciated that, in the illustrated embodiment, the lid stock attachment to first surface 112 prevents leakage of the liquid from the package; and the second surface keeps the liquid from reaching the gripping surface of the holder 130. That is, the gripping surface is disposed so as to be protected from the liquid. It will be appreciated that holder 130 is configured so as not to permit transmission of the liquid through the holder to the gripping surface (e.g., no holes or conduits permitting fluid flow to the gripping surface).

Either one or both of the attachments of the lid stock to the first surface and the second surface may form a sterile barrier. However, it will be appreciated that the attachment at the first barrier will typically be sterile since it prevents communication of the outside environment with the inside of the container where the IOL (shown in FIG. 1) is located; and the attachment at the second barrier, which prevents fluid from contacting the gripping surface of the handle, will typically only be a barrier to moisture and not a sterile barrier. It may be advantageous, in some embodiments, if the second barrier is a sterile barrier. It will be appreciated that handle 130 is disposed under the lid stock thereby maintaining the handle in a sterile state. The handle 132 may or may not contact the lid stock and may or may not be attached to the lid stock.

It will be appreciated that liquid is prevented from reaching the gripping surface 134 (shown in FIG. 2) by a combination of the second barrier and holder 130. Liquid may contact lid stock 140 at a gap 175 between holder 130 and container 110.

The container and holder may be made of any suitable material (e.g., polypropylene). The lid stock may comprise any suitable material or materials that are suitable for forming a seal with the container. The lid stock may be connected to the container and holder using any suitable sealing technology (e.g., heat, RF or adhesive). Technologies for forming liquid barriers or sterile barriers are known. For example, if the container and holder are made of polypropylene, the lid stock may comprise a foil with a film of polypropylene deposited thereon such that heat or RF energy can be applied to attach (i.e., seal) the lid stock with the container and holder.

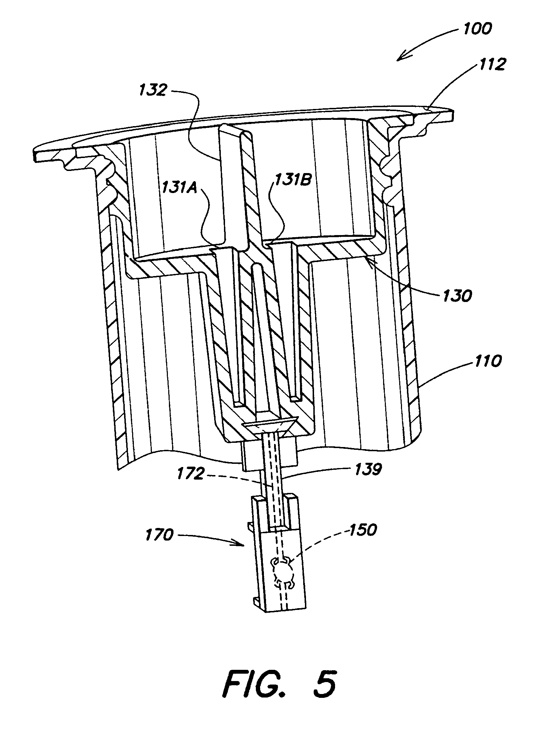

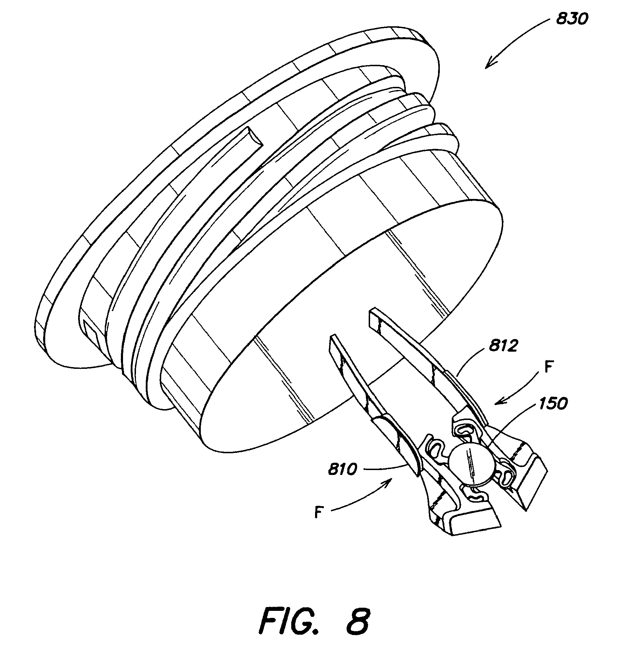

Further details of the holding mechanism are given below with reference to FIGs. 5 and 8. However, it is to be appreciated that the IOL may be directly connected to the holder (as shown in FIG. 8) or may be indirectly coupled to the holder via one or more intermediary components (as shown in FIG. 5). Whether directly connected to the holder or coupled via one or more components, the holder is coupled to the IOL so that the IOL can be lifted out of the container by removing the holder from the container.

FIG. 5 is a cutaway cross section view of the IOL package 100 of FIG. 1. As mentioned above, package 100 includes a holder 130 with a handle 132 and an intermediary component 139 which maintains IOL 150. In some embodiments, the intermediary component forms a part of an IOL injector. In FIG. 5, the intermediary component is a shuttle 170 which maintains IOL 150 in an unstressed (i.e., non-folded) state. The shuttle may be releasably attached to the holder using any suitable technique (e.g., interference fit, slid fit, compression fit).

As discussed below, the shuttle is attached to one or more injector components when the injector is prepared for injection of the IOL into a patient's eye. In the illustrated embodiment, a lumen 172 extends through the shuttle so that a plunger (shown in FIG. 6) of the injector can move the IOL from the shuttle through an inserter lumen (shown in FIG. 6) and into a patient's eye. Although it may be desirable that the intermediary component be a part of the injector it need not be so. Holder 130 includes inlets 131 a and 131 b to facilitate molding of the holder.

Referring again to FIG. 2, the heights of first surface 112 and second surface 138 may be same (i.e., the first surface and the second surface are on a common plane) or different. Typically, the heights of the surfaces will be selected to facilitate attachment (e.g., a liquid barrier attachment or a sterile barrier attachment) of the lid stock. As illustrated in FIG. 3, holder 130 and container 110 may be provided with threads 114 and 133, respectively, so that holder screws into the container. Such an arrangement may aid in retaining the fluid in the vial and maintaining sterility but is not necessary.

FIG. 6 is a schematic illustration showing an example of an inserter 500 comprising a distal portion 510, a proximal portion 520 and shuttle 170. In FIG. 5, shuttle 170 is being loaded into an IOL injector. In FIG. 6, shuttle 170 is loaded into distal portion 510 of the injector. The distal portion includes a lumen 514 extending to an opening 512 through which the IOL is injected into a patient's eye. After the shuttle is loaded into distal portion, the holder is detached from the shuttle, and a proximal portion (including a lumen 522 and a plunger 530) is attached to the distal portion. Upon attachment of the proximal portion and the distal portion, the lumens of the distal portion and the proximal portion and the shuttle align such that actuation of the plunger causes the IOL to be ejected through opening 512. Although one example of an injector for use with packages according to aspects of the invention is shown, packages as described herein may be used to load IOLs into injectors of any suitable type and in any suitable manner. In some embodiments, a holder is used to load the shuttle into the proximal portion of an injector, and a distal portion is then connected to the proximal portion before actuation of the plunger to inject the IOL occurs.

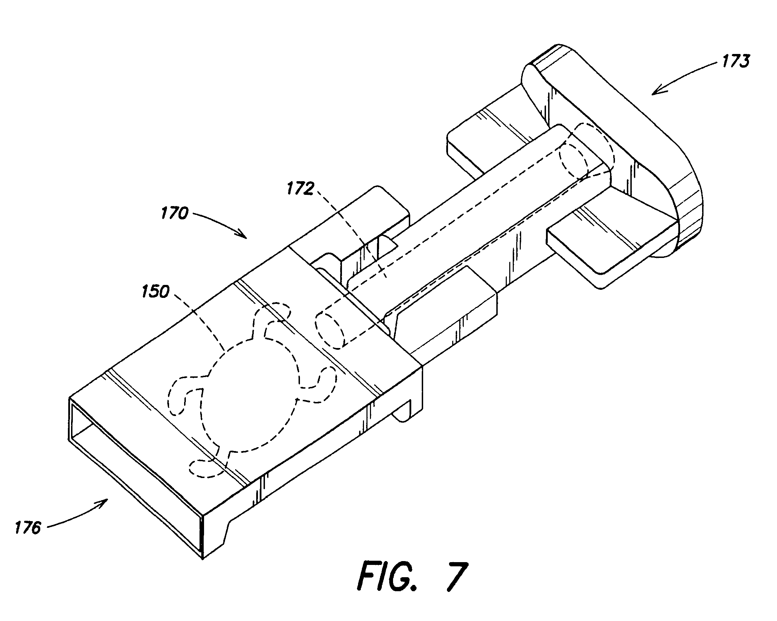

FIG. 7 is a schematic illustration showing shuttle 170 in greater detail. Shuttle 170 includes an IOL 150 and a lumen 172 extending therethrough. During injection, tip 532 (shown in FIG. 6) of the plunger enters the proximal end 173 of the lumen and moves the IOL out of the distal end 176 of the shuttle and into the distal portion 510 (shown in FIG. 6) of the injector.

FIG. 8 is a schematic illustration of an alternative embodiment of an IOL holder 830 for use with a container in IOL packages according to aspects of the invention. For example, holder 830 may be substituted for holder 130 in the package of FIG. 1 so as to be combined with a container and a lid stock in the manner described above.

Holder 830 is directly connected to the IOL without an intermediary structure. In such embodiments, the IOL may be placed into an injector portion using the holder in preparation for subsequent ejection of the IOL from the injector into a patient's eye. The holder may constitute a lens folder device to permit folding of the lens prior to loading of the lens into an injector. For example, forces F may be applied to presses 810 and 812 as indicated in FIG. 8 to fold the lens prior to or during loading.

Having thus described the inventive concepts and a number of exemplary embodiments, it will be apparent to those skilled in the art that the invention may be implemented in various ways, and that modifications and improvements will readily occur to such persons. Thus, the embodiments are not intended to be limiting and presented by way of example only. The invention is limited only as required by the following claims.

## Claims

1. An IOL package, comprising:
a container (110) comprising a first surface (112), containing a liquid inside (120);
a holder (130), comprising a second surface (138) and a handle (132) with a gripping surface,
an IOL held inside said container by said holder; and
a lid stock (140) attached to said first surface and said second surface, so as to form a first liquid barrier with said container and a second liquid barrier with said holder, whereby the gripping surface is protected from the liquid, **characterised in that** the holder comprising a recess (136) and said handle is formed in said recess; and **in that** the handle is disposed under the lid stock. (cancelled)

2. The IOL package of claim 1, wherein said first liquid barrier is a sterile barrier.

3. The IOL package of claim 1, wherein the container and holder are configured such the liquid can contact the lid stock.

4. The IOL package of claim 1, wherein the IOL is directly connected to the holder.

5. The IOL package of claim 1, wherein the IOL is coupled to the holder by one or more intermediary components.

6. The IOL package of claims 5, wherein the one or more intermediary components comprise a shuttle having a lumen extending therethrough.

7. The IOL package of claim 6, wherein the IOL is disposed in the shuttle in an unstressed state.

## Patentansprüche

1. Verpackung für Intraokularlinsen mit:
einem im Innern eine Flüssigkeit (120) enthaltenden Behälter (110) mit einer ersten Oberfläche (112);
einer Haltevorrichtung (130) mit einer zweiten Oberfläche (138) und einem Griff (132) mit einer Grifffläche;
einer im Inneren des Behälters durch die Haltevorrichtung gehaltenen Intraokularlinse; und
einem Deckel (140), der an der ersten und der zweiten Oberfläche befestigt ist, um eine erste Flüssigkeitsbarriere gegenüber dem Behälter und eine zweite Flüssigkeitsbarriere gegenüber der Haltevorrichtung zu bilden, wobei die Grifffläche vor der Flüssigkeit geschützt ist,
**dadurch gekennzeichnet, dass** die Haltevorrichtung eine Aussparung (136) umfasst und der Griff in dieser Aussparung gebildet ist, und
dass der Griff unter dem Deckel angebracht ist.

2. Intraokularlinsenverpackung nach Anspruch 1, wobei die erste Flüssigkeitsbarriere eine sterile Barriere ist.

3. Intraokularlinsenverpackung nach Anspruch 1, wobei der Behälter und die Haltevorrichtung so ausgestaltet sind, dass die Flüssigkeit mit dem Deckel in Berührung kommen kann.

4. Intraokularlinsenverpackung nach Anspruch 1, wobei die Intraokularlinse direkt mit der Haltevorrichtung verbunden ist.

5. Intraokularlinsenverpackung nach Anspruch 1, wobei die Intraokularlinse durch ein oder mehrere Zwischenelemente an die Haltevorrichtung gekoppelt ist.

6. Intraokularlinsenverpackung nach Anspruch 5, wobei das eine oder die mehreren Zwischenelemente ein Shuttle enthält/enthalten, das ein sich durch dieses erstreckendes Lumen (Hohlraum) aufweist.

7. Intraokularlinsenverpackung nach Anspruch 6, wobei die Intraokularlinse in dem Shuttle in einem entspannten Zustand angeordnet ist.

## Revendications

1. Boîtier pour lentille intraoculaire, comprenant :
un récipient (110) comprenant une première surface (112), contenant un liquide à l'intérieur (120) ;
un support (130), comprenant une deuxième surface (138) et une poignée (132) avec une surface de préhension ;
une lentille intraoculaire maintenue à l'intérieur dudit récipient par ledit support ; et
un couvercle (140) fixé à ladite première surface et à ladite deuxième surface, de manière à former une première barrière au liquide avec ledit récipient et une deuxième barrière au liquide avec ledit support, moyennant quoi la surface de préhension est protégée du liquide, **caractérisé en ce que** le support comprend un évidement (136) et ladite poignée est formée dans ledit évidement, et **en ce que** la poignée est disposée sous le couvercle.

2. Boîtier pour lentille intraoculaire selon la revendication 1, dans lequel ladite première barrière au liquide est une barrière stérile.

3. Boîtier pour lentille intraoculaire selon la revendication 1, dans lequel le récipient et le support sont configurés de sorte que le liquide peut venir en contact avec le couvercle.

4. Boîtier pour lentille intraoculaire selon la revendication 1, dans lequel la lentille intraoculaire est directement reliée au support.

5. Boîtier pour lentille intraoculaire selon la revendication 1, dans lequel la lentille intraoculaire est couplée au support par un ou plusieurs composants intermédiaires.

6. Boîtier pour lentille intraoculaire selon la revendication 5, dans lequel lesdits un ou plusieurs composants intermédiaires comprennent une navette ayant une lumière s'étendant à travers celle-ci.

7. Boîtier pour lentille intraoculaire selon la revendication 6, dans lequel la lentille intraoculaire est disposée dans la navette dans un état non contraint.
